Europäisches Patentamt

European Patent Office

Office européen des brevets

(11) Veröffentlichungsnummer: **0 119 990**
**B1**

(19)

(12) **EUROPÄISCHE PATENTSCHRIFT**

(45) Veröffentlichungstag der Patentschrift: 23.05.90

(21) Anmeldenummer: **84890049.4**

(22) Anmeldetag: **15.03.84**

(51) Int. Cl.⁵: **A 61 K 37/02,** A 61 K 35/16, A 61 K 39/395, A 61 K 37/64, A 61 L 2/04

(54) **Verfahren zur Herstellung von therapeutisch verabreichbaren, in Endbehältern abgefüllten Plasmaderivaten.**

(30) Priorität: **16.03.83 AT 932/83**

(43) Veröffentlichungstag der Anmeldung:
**26.09.84 Patentblatt 84/39**

(45) Bekanntmachung des Hinweises auf die Patenterteilung:
**23.05.90 Patentblatt 90/21**

(84) Benannte Vertragsstaaten:
**AT BE CH DE FR GB IT LI NL SE**

(56) Entgegenhaltungen:
**EP-A-0 101 935**
**FR-A-2 460 137**
**US-A-4 379 085**

Die Akte enthält technische Angaben, die nach dem Eingang der Anmeldung eingereicht wurden und die nicht in dieser Patentschrift enthalten sind.

(73) Patentinhaber: **IMMUNO Aktiengesellschaft für chemisch-medizinische Produkte Industriestrasse 72 A-1220 Wien (AT)**

(72) Erfinder: **Philapitsch, Anton Hans Kudlichgasse 5 A-2490 Ebenfurt (AT)**
Erfinder: **Linnau, Yendra, Dr. Lavendlgasse 24 A-1224 Wien (AT)**

(74) Vertreter: **Wolfram, Gustav, Dipl.-Ing. Schwindgasse 7 P.O. Box 205 A-1041 Wien (AT)**

(56) Entgegenhaltungen:
**BIOLOGICAL ABSTRACTS, Band 70, Nr. 5, 1980, Seite 2984, Zusammenfassung Nr. 28455, Biological Abstracts Inc., Philadelphia, US; P. VAN DER STARRE et al.: "Inhibition of the hypotensive effect of plasma protein solutions by C1-esterase inhibitor"**

Courier Press, Leamington Spa, England.

EP 0 119 990 B1

## EP 0 119 990 B1

56 Entgegenhaltungen:
CHEMICAL ABSTRACTS, Band 94, Nr. 3, 19.
Januar 1981, Seite 294, Zusammenfassung Nr.
13907s, Columbus, Ohio, US; H. GOERING et al.:
"Studies on the levels of C1 esterase inhibitor in
coagulation-active plasma preparations"

CHEMICAL ABSTRACTS, Band 79, Nr. 12, 24.
September 1973, Seite 248, Zusammenfassung
Nr. 70133a, Columbus, Ohio, US; E.F.
VOGELAAR et al.: "Preparation of human C1
esterase inhibitor concentrate and its possible
clinical use"

The New England J. of Med., 31/05/79, page
1276
Cardiov. Surg., J. Thorne, 79,198079, page 127

# EP 0 119 990 B1

**Beschreibung**

Die Erfindung betrifft ein Verfahren zur Herstellung von therapeutisch verabreichbaren, in Endbehältern abgefüllten Plasmaderivaten, die frei sind von Prekallikreinaktivator-Aktivität und von Aktivität hypotensiv wirksamer Bestandteile und sonstiger unerwünschter pharmakologisch aktiver Substanzen, durch stufenweise Anreicherung der Plasmaproteine und Sterilfiltration.

Die Verabreichung von Blutderivaten der bezeichneten Art kann bei Patienten zu unerwünschten Nebenreaktionen führen, insbesondere zu einem spontanen Blutdruckabfall, der gefährliche Formen annehmen kann. Diese hypotensive Wirkung der Präparate wird einem Gehalt an Proteinen zugeschrieben, die im Laufe der Plasmafraktionierung nur teilweise und mit hohen Verlusten an den erwünschten Wirksubstanzen eliminert werden können (vgl. z.B. The New England Journal of Medicine, "Hypotension Associated with Prekallikrein Activator (Hageman-Factor Fragments) in Plasma Protein Fraction", Vol. 299, Alving et al., Juli 1978, Seiten 66 bis 70). Die unerwünschten Substanzen mit hypotensiver Wirkung wurden einerseits als Fragmente des Gerinnungsfaktors XII (F XII$_f$) klassifiziert und ihre Aktivität als "Prekallikreinaktivator-Aktivität" (PKKA) bezeichnet; anderseits durch Substanzen, die als Verunreinigungen in den Präparaten pharmakologisch unerwünschte Nebenreaktionen hervorrufen können und unabhängig von PKKA-Wirkung Reaktionen hervorrufen. Die in-vitro-Testung der PKKA-Aktivität erfolgt üblicherweise über die Bestimmung von Kallikrein (KK), das nach Zusatz von F XII$_f$ aus einer inaktiven Vorstufe (Prekallikrein) entsteht. Kallikrein spaltet chromogene Substrate auf Tripeptidbasis. Die dabei freigesetzte chromophore Gruppe wird photometrisch vermessen (vgl. Develop. biol. Standard, Vol. 44, Seiten 115 bis 120, "The Assay of Prekallikrein-Activator in Human Blood Products", T. J. Snape et al.). Es wird indirekt aus den Gehalt von PKKA im Testmaterial geschlossen und in bezug auf einen Internationalen Standard (Reference PKA Standard Lot 2 es Bureau of Biologics, Bethesda, USA) gesetzt.

Da Nebenreaktionen nicht nur von PKKA-Gehalt abhängig sind, werden zur Prüfung von Blutderivaten weitere Testmethoden angewendet, die auf in-vivo-Reaktionen schließen lassen. Eine dieser Methoden ist der Kallikrein-burst-Test, bei dem die Probe zu humanem Plasma zugesetzt wird und die dabei spontan auftretende Kallikreinfreisetzung im Plasma mit chromogenem Substrat S 2302 (Kabi) über die Freisetzung von p-Nitroanilid aus dem chromogenen Substrat photometrisch gemessen wird. Diese Kallikreinfreisetzung aus Plasma ist im Gegensatz zur PKKA induzierten Kallikreinfreisetzung aus der inaktiven Vorstufe unterschiedlich zu bewerten, da sie keine Kinetik darstellt, sondern spontan innerhalb der ersten Minuten auftritt und wieder verschwindet.

Eine weitere Möglichkeit, pharmakologische Nebenreaktionen zu testen, ist die Kontraktion am isolierten Ileum von Meerschweinchen. Durch die zu testenden Proben werden in humanem Plasma Substanzen freigesetzt, die zu einer Kontraktion am isolierten Ileum führen.

Es ist bekannt, daß die Aktivität des Prekallikreinaktivators reduziert bzw. unterdrückt werden kann, wenn ein Inhibitor, nämlich der C$_1$-Esterase-Inhibitor (C$_1$INA), zugegen ist. Dieser C$_1$-Esterase-Inhibitor ist in der Literatur auch als F XII$_f$-Inhibitor beschrieben (vgl. The Journal of Clinical Investigation, Vol. 52, Juni 1973, Seiten 1402—1409, A. D. Schreiber et al.). Der Inhibitor wird aus Humanplasma nach bekannten Verfahrensweise präpariert (Vox Sang. 26: 118—127 (1974), "Contributions to the Optimal Use of Human Blood", E. F. Volgelhaar et al.).

Es wurde bereits vorgeschlagen, C$_1$-Esterase-Inhibitor unmittelbar vor der Applikation am Patienten einer stabilen Plasmaproteinlösung (PPL) zuzugeben. Diese fertig applizierbare Zubereitung hat eine Ionenstärke von 130 bis 160 mequ/l (vgl. The New England Journal of Medicine, Mai 31, Seite 1276 (1979) und J. Thorac Cardiov. Surg. 79, Seiten 738—740 (1980). Ein Nachteil dieser Methode liegt jedoch darin, daß der C$_1$-Esterase-Inhibitor nur unzureichend ausgenutzt wird.

Die Erfindung bezweckt die Vermeidung dieses Nachteiles und stellt sich darüber hinaus die Aufgabe, nicht nur Albuminfraktionen (PPL), frei von Preikallikreinaktivator-Aktivität, sondern auch andere Plasmafraktionen zur Verfügung zu stellen, die neben der Freiheit von Prekallikreinaktivator-Aktivität auch frei sind von preikallikrein-Burst-aktiven und ileakontrahierenden Substanzen; sie sollen also keinerlei pharmakologisch negative Nebenwirkungen bei der Applikation entfalten und sie sollen überdies mit verbesserter Wirkung und Ausbeute herstellbar sein.

Diese Aufgabe wird erfindungsgemäß dadurch gelöst, daß während des Herstellungsprozesses des Plasmaderivates C$_1$-Esterase-Inhibitor zugesetzt wird, um einen thermisch stabilen PKKA/C$_1$INA-Komplex auszubilden, das Plasmaderivat Endbehälter abgefüllt wird und diese einer Virushitzeinaktivierung unterworfen werden.

Nach einer bevorzugten Ausführungsform der Erfindung wird der Zusatz des C$_1$-Esterase-Inhibitors bei einer Na$^+$-Konzentration von 5 bis 200 mequ/l, vorzugsweise 10 bis 50 mequ/l, und in einem pH-Wert-Bereich von 5 bis 9 durchgeführt. Es hat sich gezeigt, daß der Zusatz des C$_1$-Esterase-Inhibitors bei niedriger Salzkonzentration eine bessere Ausnützung des Inhibitors bei der Bildung des Komplexes PKKA/C$_1$INA ermöglicht. Eine weitere bevorzugte Ausführungsform bei der Herstellung von Albuminpräparaten besteht darin, daß nach der Abfüllung des Produktes in die Endbehälter diese einer Virushitzeinaktivierung während 10 Stunden bei 60°C unterworfen werden. Diese Ausführungsform beruht auf der überraschenden Erkenntnis, daß — wenngleich der C$_1$-Esterase-Inhibitor hitzelabil ist — die Komplexverbindung des Preikallikreinaktivators mit dem C$_1$-Esterase-Inhibitor hitzestabil ist und einer Hitzeinaktivierung — üblicherweise einer Hitzeinaktivierung während 10 Stunden bei 60°C — standhält. Eine weitere bevorzugte

3

Ausführungsform besteht darin, daß der zugesetzte $C_1$-Esterase-Inhibitor selbst virusinaktiviert ist.

Die Testmethoden für die Nebenwirkungsfreiheit der erfindungsgemäß hergestellten Plasmaderivate sind wie folgt durchzuführen:

Bestimmung des Prekallikreinaktivators:

**1. Methode:**

Aus einer gereinigten Prekallikrein-Präparation (PKK) wird mittels eines Prekallikreinaktivators (PKKA) Kallikrein (KK) generiert. Das Kallikrein spaltet amidolytisch p-Nitroanilid (pNA) von einem spezifischen chromogenen Substrat ab. Die Konzentration von pNA wird photometrisch bei einer Wellenlänge von 405 nm gemessen.

**2. Reagentien:**

Puffer I: 6,60 g TRIS und 23,38 g NaCl werden in ca. 500 ml $H_2O$ dest. gelöst und mit verdünnter HCl auf einen pH-Wert von 8,0 eingestellt und mit $H_2O$ dest. auf 1000 ml aufgefüllt.

Puffer II: 1,81 g TRIS, 1,02 g Imidazol und 6,43 g NaCl werden in ca. 500 ml $H_2O$ dest. gelöst und mit verdünnter HCl auf einen pH-Wert von 7,9 gestellt und mit $H_2O$ dest. auf 1000 ml aufgefüllt.

Chromogenes Substrat: S 2302 (Kabi) H-D-Prolyl-L-phenylalanyl-L-arginin-p-nitroaniliddihydrochlorid. Eine 10 m-molare wässerige Lösung wird hergestellt. 25 mg S 2302 in 4,1 ml $H_2O$ dest.

Prekallikrein-Präparation: Die Herstellung der Präparation erfolgt nach einer Vorschrift von Harpel, modifiziert von M.S. Horowitz (New York Blood Center). Dabei wird humanes Citratplasma mit Hilfe einer DEAE-Cellulose behandelt. Die nicht an DEAE-Cellulose gebundene Fraktion enthält das Prekallikrein.

Positive Kontrolle (Standard): Als Standard (= Bezugswert) wird eine Albuminpräparation des Bureau of Biologics (BoB) der Food and Drug Administration, Bethesda, Maryland 20205, USA, verwendet. Diese Präparation enthält einen Prekallikreinaktivator. Die Kallikreingeneration mit diesem BoB-Standard stellt den Bezugswert 1 dar bzw. wird gleich 100% gesetzt.

Probe: Die Probe wird, wenn erforderlich, gelöst bzw. verdünnt in dem Test eingesetzt.

Test: In einem Wasserbad bei einer Temperatur von 37° C werden in ein Plastikröhrchen

    100 µl Prekallikreinpräparation
      50 µl Puffer I
      25 µl Probe

pipettierrt. Nach einer Inkubationszeit von 15 min bei

    37°C werden
    300 µl Puffer II
      50 µl S 2302 Substrat

pipettiert. Diese Mischung wird in ein auf 37°C temperiertes Photometer eingebracht und die Zunahme der optischen Dichte pro Minute ($\Delta$OD/min) bei einer Wellenlänge von 405 nm, bei einer Schichtdicke von 10 mm, gemessen. Die Aktivität einer Probe ($\Delta$OD/min) wird faktoriell — gegenüber dem BoB-Standard mit der Zahl 1-bzw. in % vom BoB-Standard ausgedrückt.

Bestimmung der spontanen Kallikrein-Freisetzung im Plasma (Burst-Reaktion):

**1. Methode:**

Aus humanem Plasma wird mittels der zugefügten Probe spontan (in der ersten Minute) Kallikrein (KK) freigesetzt. Kallikrein spaltet amidolytisch pNA von dem spezifischen chromogenen Substrat S 2302 ab. pNA wird photometrisch bei 405 nm gemessen.

**2. Reagentien:**

a) Humanes Plasma aus einem Normalspenderkollektiv

b) Puffer II, chromogenes Substrat wie unter 'Bestimmung d. PKKA" beschrieben

c) Probe wird unverdünnt oder in genau festgesetzten Verdünnungen dem Testgemisch zugesetzt.

Um die spontan gebildeten Kallikreinaktivitäten besser nachweisen zu können, d. h. die Nachweisgrenzen zu senken, kann dem humanen Plasma noch $C_1$INA Antiserum zugesetzt werden, des den Hauptinhibitor der Kallikrein-Burst-Aktivität neutralisiert. Damit wird erreicht, daß die Aktivitätshöhe des Burst-Testes gesteigert wird. Weiters kann die zugesetzte Probe Enzyme enthalten, die mit dem chromogenen Substrat selbst reagieren. Dem kann entgegengewirkt werden, daß mit bestimmten Inhibitoren diese autoamidolytische Aktivität gesenkt wird, wobei selbst die Kallikrein-Burst-Aktivität nicht beeinflußt wird (z. B. Aprotinin, Trasylol).

**3. Test:**

In ein Plastikröhrchen bei 37°C werden

    0,25 ml humanes Plasma
    0,025 ml Probe pipettiert und 1 min bei 37°C inkubiert, anschließend werden sofort

    0,25 ml Puffer II
    0,05 ml chromogenes Substrat S 2302

zugefügt, diese Mischung in ein auf 37°C temperiertes Photometer eingebracht und die Zunahme der optischen Dichte pro Minute (ΔOD/min) bei einer Wellenlänge von 405 nm in 10 mm Schichtdicke gemessen.

Bestimmung hypotensiv wirksamer Substanzen am isolierten Meerschweinchenileum:

1. Methode:

Aus humanen Plasma werden mittels zu testender Probe Substanzen freigesetzt, die auf glatter Muskulatur Reaktionen hervorrufen (Handbook of Experimental Pharmacology, Edt. E. G. Erdös, Vol. XXV, Springer 1970). Damit die freigesetzten reaktiven Substanzen nicht abgebaut werden und sich dadurch der Testung am Ileum entziehen, wird dem Reaktionsgemisch ein geeigneter Kininase-Inhibitor (z. B. D-3-Mercapto-2-methylpropionyl-L-Prolin) zugesetzt.

2. Test:

Ein isoliertes Merschweinchenileum mit einer Länge von 20 mm und einem Durchmesser von 5 mm wird in ein 10 ml fassendes Organbad (modifiziert nach Schulz-Dahle) eingebracht und mit etwa 10 aufeinanderfolgenden Histaminzugaben von je 5 ng Histamin auf seine Kontraktionsfähigkeit geeicht. Anschließend wird das Ileum von Histamin freigewaschen.

In einem Wasserbad von 37°C wird ein Reaktionsgemisch nach folgenden Pipettierfolge erstellt:

100 µl humanes Plasma
250 µl D-3-Mercapto-2-methylpropionyl-L-Prolin entsprechend 2 µg
440 µ zu testende Probe

werden gemischt und 10 Minuten bei 37°C inkubiert. Anschließend wird dieses Reaktionsgemisch in das Organbad auf das Ileum gebracht. Wenn durch den Probezusatz reaktive Substanzen freigesetzt werden, kontrahiert das Ileum. Als Maß für die Kontraktion wird die Amplitude, die innerhalb von 30 Sekunden nach Zugabe des Reaktionsgemisches auf das Ileum erreicht wird, in Millimeter gemessen, wobei die Eichung mit 0,315 mN pro mm Amplitude bei einer Meßverstärkung von 5 mV über eine Skalenlänge von 250 mm festgelegt ist.

Das erfindungsgemäße Verfahren wird durch folgende Beispiele näher erläutert.

## Beispiel 1

Herstellung einer Albuminfraktion:

Zu 10 l menschlichem Blutplasma werden bei einem pH Wert von 7,0 und einer Temperatur von −2°C, 8% Äthanol zugesetzt, wobei ein Niederschlag, der Fibrinogen enthält, ausfällt. Nach Abtrennen dieses Niederschlages wird die Äthanolkozentration auf 25% erhöht und die Temperatur auf −6°C gesenkt. Der ausfallende Niederschlag, der Immunglobulin enthält, wird abgetrennt und die Äthanolkonzentration des Überstandes wird auf 40% erhöht, bei einem pH-Wert von 6,5 und einer Temperatur von −8°C.

Der gebildete Niederschlag wird abgetrennt und verworfen. Der pH-Wert des Überstandes wird auf 5,4 gestellt, bei gleicher Temperatur; dabei fällt Albumin aus. Dieses wird durch Zentrifugieren abgetrennt und einem weiteren Reinigungsschritt unterworfen: der Niederschlag wird in Wasser gelöst und die Äthanolkonzentration bei einem pH-Wert von 4,8 und einer Temperatur von −2°C und 10% gestellt. Das ausgefallene Globulin wird abgetrennt und verworfen. Die Äthanolkonzentration des Überstandes wird auf 40% erhöht, die Temperatur auf −8°C gesenkt und der pH-Wert auf 5,1 gestellt.

Der Albuminniederschlag wird durch Zentrifugieren gesammelt und gegebenenfalls nach zwischenzeitlicher Lyophilisierung erfindungsgemäß in folgender Weise weiterbehandelt:

Das Albuminkonzentrat wird in NaCl-Lösung gelöst, wobei eine 4,3 %ige Proteinlösung mit einem pH-Wert von 6,9 und einer Salzkonzentration von 140 mequ $Na^+/1$ erhalten wird. Teilmengen der Lösung werden mit $C_1INA$ versetzt, um eine Konzentrationsreihe von

1. 0,01 E $C_1INA$ pro g Protein
2. 0,1 E $C_1INA$ pro g Protein
3. 1,0 E $C_1INA$ pro g Protein
4. 10,0 E $C_1INA$ pro g Protein

zu erhalten. Die Mischungen bleiben etwa 24 Stunden bei Raumtemperatur stehen. Anschließend werden den Lösungen Stabilisatoren (Na-Caprylat und Na-Acetyltryptophanat) in üblicher Weise zugesetzt, sterilfiltriert und 10 Stunden bei 60°C erhitzt. Als Kontrolle dient eine parallel behandelte Albuminprobe der gleichen Charge ohne Zusatz von $C_1INA$.

Eine Einheit $C_1$-Inhibitor (E) entspricht der Menge $C_1$-Inhibitor, die in 1 ml Frischplasma enthalten ist.

EP 0 119 990 B1

| | PKKA-Gehalt in % vom BoB-Standard | Burst-Reaktion 1000 $\Delta$OD/min | Ileumreaktion in mm der Kontraktionsamplitude |
|---|---|---|---|
| Albuminlösung nicht erhitzt | 119 | 19 | 55 |
| Albuminlösung ohne $C_1$ INA-Zusatz | 68 | 18 | 22,5 |
| Albuminlösung mit 0,01 E $C_1$ INA/g Protein | 64 | 14 | 21,3 |
| Albuminlösung mit 0,1 E $C_1$ INA/g Protein | 43 | 10 | 21 |
| Albuminlösung mit 1,0 E $C_1$ INA/g Protein | 4 | 0 | 0 |
| Albuminlösung mit 10,0 E $C_1$ INA/g Protein | 0 | 0 | 0 |

10 Stunden bei 60° C erhitzt

Beispiel 2

Herstellung einer Albuminfraktion:

Eine aus der Alkoholfraktionierung — wie unter Beispiel 1 beschrieben — anfallende Albuminfraktion wird wässerig gelöst, wobei eine Lösung mit 5% Proteingehalt und eine $Na^+$-Konzentration von etwa 10 mequ/1 erhalten wird. Der pH-Wert der Lösung beträgt 7,2.

Diese Lösung wird mit $C_1$INA versetzt, um eine Konzentrationsreihe von

1. 0,01 E $C_1$INA pro g Protein
2. 0,1 E $C_1$INA pro g Protein
3. 1,0 E $C_1$INA pro g Protein
4. 10,0 E $C_1$INA pro g Protein

zu erhalten. Die Mischungen bleiben etwa 24 Stunden bei 37°C stehen, werden anschließend mit Stabilisatoren, wie in Beispiel 1 angegeben, versetzt, auf eine $Na^+$-Konzentration von 130 bis 160 mequ/1 gebracht, sterilfiltriert und 10 Stunden bei 60°C erhitzt.

EP 0 119 990 B1

10 Stunden bei 60 °C erhitzt

| | PKKA-Gehalt in % vom BoB-Standard | Burst-Reaktion 1000 $\Delta$OD/min | Ileumreaktion in mm der Kontraktionsamplitude |
|---|---|---|---|
| Albuminlösung nicht erhitzt | 210 | 27 | O |
| Albuminlösung mit 0,01 E $C_1$INA/g Protein | 24 | 5 | O |
| Albuminlösung mit 0,1 E $C_1$INA/g Protein | 0,6 | 2 | O |
| Albuminlösung mit 1,0 E $C_1$INA/g Protein | O | O | O |

## Beispiel 3

Herstellung einer Immunglobulinfraktion:

Zu 10 l menschlichem Blutplasma werden bei einem pH-Wert von 7,0 und einer Temperatur von −2°C, 8% Äthanol zugesetzt, wobei ein Niederschlag, der Fibrinogen enthält, ausfällt. Nach Abtrennen dieses Niederschlages wird die Äthanolkonzentration auf 25% erhöht und die Temperatur auf −6°C gesenkt. Der ausfallende Niederschlag, der im wesentlichen aus Immunglobulin besteht, wird in einem Phosphat-Acetat-Puffer suspendiert und mit 12% Äthanol bei einem pH-Wert von 5,3 und einer Temperatur von −2°C versetzt. Der ausfallende Niederschlag, der $\alpha$- und $\beta$-Globulin enthält, wird verworfen; sodann die Äthanol-Konzentration des Überstandes bei einem pH-Wert von 7,0 und einer Temperatur von −6°C auf 25% erhöht, wodurch Immunglobulin ausgefällt wird. Das so erhaltene Immunglobulin wird gesammelt, gegebenenfalls gefriergetrocknet und erfindungsgemäß in folgender Weise weiterbehandelt:

Die Lösung mit einem Proteingehalt von 12,5% unter physiologischen Salz- und pH-Bedingungen wird mit $C_1$INA versetzt, so daß sich ein Verhältnis von

1. 0,01 E $C_1$INA pro g Protein
2. 0,1 E $C_1$INA pro g Protein
3. 1,0 E $C_1$INA pro g Protein
4. 10,0 E $C_1$INA pro g Protein

ergibt. Die Mischungen verbleiben etwa 24 Stunden bei +4°C, werden sterilfiltriert und abgefüllt.

| | PKKA-Gehalt in % vom BoB-Standard | Burst-Reaktion 1000 $\Delta$OD/min | Ileumreaktion in mm der Kontraktionsamplitude |
|---|---|---|---|
| Immunglobulin ohne C$_1$INA-Zusatz | 3600 | 204 | 88,8 |
| Immunglobulin mit 0,01 E C$_1$INA/g Protein | 2300 | 154 | 72,5 |
| Immunglobulin mit 0,1 E C$_1$INA/g Protein | 2200 | 151 | 50 |
| Immunglobulin mit 1,0 E C$_1$INA/g Protein | 130 | 9 | 0 |
| Immunglobulin mit 10,0 E C$_1$INA/g Protein | 0 | 0 | 0 |

EP 0 119 990 B1

## Beispiel 4

Herstellung einer Immunglobulinfraktion:

Eine wie in Beispiel 3 hergestellte Immunglobulinfraktion mit einem Proteingehalt von 5,03% wird auf

1. 0,01 E $C_1$INA pro g Protein
2. 0,1 E $C_1$INA pro g Protein
3. 1,0 E $C_1$INA pro g Protein
4. 10,0 E $C_1$INA pro g Protein

gestellt, sterilfiltriert, abgefüllt und 24 Stunden bei 37°C inkubiert.

| | PKKA-Gehalt in % vom BoB-Standard | Burst-Reaktion 1000 $\Delta$OD/min | Ileumreaktion in mm der Kontraktionsamplitude |
|---|---|---|---|
| Immunglobulin ohne $C_1$INA-Zusatz | 180 | 7 | 42,5 |
| Immunglobulin mit 0,01 E $C_1$INA/g Protein | 160 | 5 | 22,5 |
| Immunglobulin mit 0,1 E $C_1$INA/g Protein | 86 | O | 7,5 |
| Immunglobulin mit 1,0 E $C_1$INA/g Protein | 5 | O | 3,8 |
| Immunglobulin mit 10,0 E $C_1$INA/g Protein | O | O | O |

EP 0 119 990 B1

Beispiel 5

Herstellung einer Faktor VIII-Fraktion:

46 l frisch gefrorenes Plasma werden bei 0°C bis +4°C aufgetaut. Das entstandene Kryopräzipitat wird durch Zentrifugieren abgetrennt und in 960 ml 0,1 %iger Tri-Natriumcitrat bei 37°C gelöst. 8% Polyäthylenglykol 2000 werden bei pH 6,3 zugesetzt. Dabei entsteht ein Niederschlag, der durch Zentrifugieren abgetrennt und verworfen wird. Durch Zugabe von 12% Äthanol zum Überstand, bei −3°C, wird der angereicherte Faktor VIII ausgefällt. Nach dem Abtrennen wird er in einem physiologischen Puffer gelöst und gegebenenfalls nach zwischenzeitlicher Lyophilisierung erfindungsgemäß weiterbehandelt:

Aus der Lösung mit einer Proteinkonzentration von 1,8% Protein wird durch Zusatz von $C_1$INA eine Konzentrationsreihe von

1. 0,01 E $C_1$INA pro g Protein
2. 0,1 E $C_1$INA pro g Protein
3. 1,0 E $C_1$INA pro g Protein

bereitet. Die Mischungen bleiben etwa 24 Stunden bei +4°C stehen; anschließend wird sterilfiltriert und ein Fertigpräparat hergestellt.

| | PKKA-Gehalt in % vom BoB-Standard | Burst-Reaktion 1000 $\Delta$OD/min | Ileumreaktion in mm der Kontraktionsamplitude |
|---|---|---|---|
| Faktor VIII-Präparation ohne $C_1$INA-Zusatz | 64 | 71 | 35 |
| Faktor VIII-Präparation mit 0,01 E $C_1$INA/g Protein | 34 | 62 | 27,5 |
| Faktor VIII-Präparation mit 0,1 E $C_1$INA/g Protein | 12 | 23 | 5 |
| Faktor VIII-Präparation mit 1,0 E $C_1$INA/g Protein | 0 | 18 | 5 |

EP 0 119 990 B1

In den weiteren Beispielen wird veranschaulicht, daß auch andere Viren als das Heptatitis-Virus bei der Herstellung von Albuminpräparaten im Rahmen der erfindungsgemäßen Verfahrens inaktivierbar sind.

Beispiel 6

Aus einer Albuminfraktion wird eine 5 %ige Plasmaproteinlösung hergestellt. Die $Na^+$-Konzentration dieser Lösung beträgt 140 mequ $Na^+$/l, der pH-Wert ist 6,9. Diese Lösung wird mit Stabilisatoren (Na-Caprylat und Na-Acetyltryptophanat) versetzt. Gleichzeitig wird $C_1$-Esterase-Inhibitor in solcher Menge zugesetzt, daß ein Gehalt von 13 Einheiten $C_1$INA/g Protein resultiert. Die so hergestellte Mischung wird sterilfiltriert und anschließend mit Poliovirus Typ I versetzt. Dann erfolgt eine Hitzeinaktivierung während 10 Stunden bei 60°C. Zur Kontrolle verbleibt eine mit Poliovirus Typ I versetzte Plasmaproteinlösung 10 Stunden bei +4°C. Die Kontrollprobe und die hitzebehandelte Lösung werden einer Virustiterbestimmung unterzogen. Die Werte in der folgenden Tabelle sind dekadische Logarithemen der $TCID_{50}$ pro 0,1 ml, wobei $TCID_{50}$ bedeutet, daß 50% der Gewebekultur-Ansätze einen cytopathogenen Effekt zeigen.

**Tabelle:**

| | |
|---|---|
| Virustiter der Kontrolle | $>$ 9 |
| Virustiter der 10 Stunden bei 60° C behandelten Probe | $<$ 1 |

Beispiel 7

Aus einer Albuminfraktion wird eine 5 %ige und eine 20 %ige Proteinlösung hergestellt. Die $Na^+$-Konzentration beider Lösungen wird auf 150 mequ/l eingestellt, der pH-Wert beträgt 7,0. Die Lösungen werden, wie in Beispiel 6 beschrieben, mit Stabilisatoren und $C_1$INA versetzt, und einer Sterilfiltration unterworfen. Anschließend werden die 5 %ige bzw. 20 %ige Proteinlösung mit Poliovirus Typ I, Rotavirus, Hundehepatitisvirus und Coxsackievirus versetzt und einer Hitzeinaktivierung über 16,5 Stunden bei 60°C unterzogen. Zur Kontrolle verbleiben mit Virus versetzte Proben die gleiche Zeit bei +4°C. Die Testung über die Virusabtötung erfolgt wie in Beispiel 6 beschreiben.

**Tabelle:**

| | Polio Typ I | Rota virus | Hunde hepatitis | Coxsackie |
|---|---|---|---|---|
| Kontrolle | 6,6 | 5,6 | 5,4 | 6,6 |
| 5 %ige Protein- lösung | $<$1 | $<$1 | $<$1 | $<$1 |
| 20 %ige Protein- lösung | $<$1 | $<$1 | $<$1 | $<$1 |

(16,5 Stunden 60° C)

**Patentansprüche**

1. Verfahren zur Herstellung von therapeutische verabreichbaren, in Endbehältern abgefüllten Plasmaderivaten, die frei sind von Prekallikreinaktivator-Aktivität und von Aktivität hypotensiv wirksamer Bestandteile und sonstiger unerwünschter pharmakologisch aktiver Substanzen, durch stufenweise Anreicherung der Plasmaproteine und Sterilfiltration, dadurch gekennzeichnet, daß während des Herstellungsprozesses des Plasmaderivates $C_1$-Esterase-Inhibitor zugesetzt wird, um einen thermisch stabilen PKKA/$C_1$INA-Komplex auszubilden, das Plasmaderivat in Endbehälter abgefüllt wird und diese einer Virushitzeinaktivierung unterworfen werden.

2. Verfahren nach Anspruch 1, dadurch gekennzeichnet, daß der Zusatz des $C_1$-Esterase-Inhibitors bei einer $Na^+$-Konzentration von 5 bis 200 mequ/l, vorzugsweise 10 bis 50 mequ/l, und in einem pH-Wert-Bereich von 5 bis 9 durchgeführt wird.

3. Verfahren nach Anspruch 1 zur Herstellung eines Albuminpräparates, dadurch gekennzeichnet, daß nach der Abfüllung des Produktes in die Endbehälter diese einer Virushitzeinaktivierung während 10 Stunden bei 60°C unterworfen werden.

15

4. Verfahren nach den Ansprüchen 1 bis 3, dadurch gekennzeichnet, daß der zugesetzte $C_1$-Esterase-Inhibitor selbst virusinaktiviert ist.

**Revendications**

1. Procédé pour la fabrication de dérivés du plasma mis dans des récipients pour l'emploi final, administrables en thérapeutique, qui sont exempts d'activité d'activateur de prékallikréine et d'activité de constituants à action hypotensive et d'autres substances pharmacologiquement actives indésirables, par concentration par étapes des protéines du plasma et filtration en conditions stériles, caractérisé en ce que pendant le procédé de fabrication du dérivé de plasma, on ajoute un inhibiteur de $C_1$-estérase pour former un complexe $PKKA/C_1INA$ thermiquement stable, on remplit des récipients finals avec le dérivé de plasma et on les soumet à une inactivation thermique des virus.

2. Procédé selon la revendication 1, caractérisé en ce que l'addition de l'inhibiteur de $C_1$-estérase est effectuée à une concentration en $Na^+$ de 5 à 200 méq/l, de préférence de 10 à 50 méq/l, et dans un domaine de pH de 5 à 9.

3. Procédé selon la revendication 1 pour la fabrication d'une préparation d'albumine, caractérisé en ce que les récipients finals sont soumis après remplissage avec le produit à une inactivation thermique des virus pendant 10 h à 60°C.

4. Procédé selon les revendications 1 à 3, caractérisé en ce que l'inhibiteur de $C_1$-estérase ajouté est lui-même soumis à l'inactivation des virus.

**Claims**

1. A method for the production of therapeutically administrable plasma derivatives filled in final storage containers, which are free from prekallikrein activator activity and from activity of hypotensively active constituents and other undesired pharmacologically active substances, by stepwise enrichment of the plasma proteins and sterile filtering, characterised in that $C_1$ esterase inhibitor is added during the production process of the plasma derivative, in order to form a thermally stable $PKKA/C_1INA$ complex, the plasma derivative is filed in final storage containers and is subjected in the same to a thermal virus inactivation.

2. A method according to claim 1, characterised in that the addition of the $C_1$ esterase inhibitor is carried out at a $Na^+$ concentration of 5 to 200 mequ/l, preferably 10 to 50 mequ/l, and in a pH range of from 5 to 9.

3. A method according to claim 1 for the production of an albumin preparation, characterised in that, after filling the product into the final storage containers, these are subjected to a thermal virus inactivation for 10 hours at 60°C.

4. A method according to claims 1 to 3, characterised in that the $C_1$ esterase inhibitor added is itself virus inactivated.